# EUROPÄISCHE PATENTANMELDUNG

(11) **EP 1 142 896 A1**
(43) Veröffentlichungstag der Anmeldung: **10.10.2001**
(21) Anmeldenummer: 01108231.0
(22) Anmeldetag: 31.03.2001
(51) Int. Cl.: C07F 7/08, C07D 251/16, C07D 251/22, C07D 251/46, C07D 251/52, C08K 5/00, C08K 5/3492, C08K 5/544, C07F 7/18

(54) **Organosiliciumtriazinverbindungen und deren Verwendung in Kautschukmischungen**

(30) Priorität: 08.04.2000 DE 10017654
(71) Anmelder: Degussa AG, 40474 Düsseldorf (DE)
(72) Erfinder: Krafczyk, Roland, Dr., 79618 Rheinfelden (DE); Luginsland, Hans-Detlef, Dr., 50968 Köln (DE); Michel, Rudolf, 63579 Freigericht (DE); Münzenberg, Jörg, Dr., 63457 Hanau (DE)

(57) **Zusammenfassung**

Organosiliciumverbindungen der allgemeinen Formel 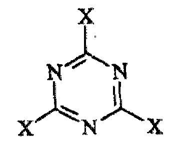 dadurch gekennzeichnet, dass die Substituenten X gleich oder verschieden sind und X eine der folgenden Gruppen A, B oder C ist: A = Y-R¹-Sₙ- mit Y = -Si(R²)₃, -Si(R²)(R³)₂ oder -Si(R²)₂ (R³) mit R² = Alkoxyrest mit 1 bis 4 C-Atomen, R³ = Alkylrest mit 1 bis 8 C-Atomen, R¹ = linear oder verzweigtem Alkyliden mit 1 bis 10 C-Atomen, n = 1 - 8 oder Gemische davon, B = OR⁴, NR⁵R⁶, SR⁷, SCN oder -CO-R⁸ mit R⁴, R⁵, R⁶, R⁷ = H, verzweigter oder unverzweigter Alkylrest mit 1 - 10 C-Atomen oder substituierter oder unsubstituierter aromatischer Rest mit 6 - 30 C-Atomen, der gegebenenfalls durch N-, S- oder O-Atome unterbrochen ist, R⁸ = linear oder verzweigter Alkylrest mit 1-20 C Atomen, C = (Sₘ)/2 mit m = 1- 8 oder Gemische davon mit der Maßgabe, daß die Gruppe C zwei Triazin Einheiten verbruckt, und im Molekul mindestens eine Gruppe A vorhanden ist und die Kombination einer Gruppe A zusammen mit zwei Mercaptogruppen beziehungsweise einer Mercaptogruppe und einer Aminogruppe NR⁵R⁶ ausgeschlossen ist. Die Organosiliciumverbindungen werden hergestellt, indem man die Triazinverbindungen mit funktionellen Gruppen (z.B. Chlor) und entsprechenden Verbindungen (z.B. Mercapto) umsetzt. Die Organosiliciumverbindungen können in Kautschukmischungen verwendet werden.

## Beschreibung

Die vorliegende Erfindung betrifft Organosiliciumverbindungen, ein Verfahren zu ihrer Herstellung und ihrer Verwendung.

Es ist bekannt, daß schwefelhaltige Organosiliciumverbindungen, wie 3-Mercaptopropyltrimethoxysilan, 3-Mercaptopropyltriethoxysilan, 3-Thiocyanatopropyltriethoxysilan oder Bis-(3-Triethoxysilylpropyl)tetrasulfan und -disulfan als Silanhaftvermittler oder Verstärkungsadditive in oxidisch gefüllten Kautschukmischungen eingesetzt werden. Die Kautschukmischungen werden unter anderem für technische Gummiartikel und für Teile des Autoreifens, insbesondere für Laufflächen, verwendet (DE 2 141 159, DE 2 212 239, US 3 978 103, US 4 048 206).

Es ist weiterhin bekannt, daß die Alkoxysilylfunktion, meist eine Trimethoxysilyl- oder Triethoxysilylgruppe, während der Mischungsherstellung mit den Silanolgruppen des Füllstoffs, meist Kieselsäure, reagiert und somit das Silan auf der Füllstoffoberfläche fixiert wird. Die Ausbildung der Füllstoff-Kautschukbindung erfolgt dann während des Vulkanisationsprozesses über die Schwefelfunktionalität des fixierten Silans.

Als besonders effektiv haben sich für diese Anwendung sogenannte geblockte Mercaptosilane erwiesen (WO99/09036). Diese Verbindungen enthalten eine polymerreaktive Monosulfanfunktion, die mit carbonylartigen Gruppen abgesättigt ist. Bei diesen carbonylartigen Blockierungsgruppen kann es sich neben Gruppen wie -C(=O)R, - C(=S)R, -C(=NR')R auch um Heterocarbonyle wie Sulfongruppen, Phosphongruppen und andere handeln. Der wesentliche Vorteil dieser Verbindungen ist, daß eine vorzeitige Reaktion der polymerreaktiven Schwefelfunktion durch gezielte Aktivierung dieser Funktion unterdrückt werden kann. Durch diese Produkte wird die Produktionssicherheit kieselsäuregefüllter Gummiartikel deutlich erhöht.

Aus der Literatur ist ferner bekannt, daß Triazine sehr wirksame Vulkanisationsbeschleuniger darstellen. Bei Einsatz dieser Verbindungen kann Nitrosaminbildung unterdrückt werden, was einen bedeutenden toxikologischen und ökotoxikologischen Vorteil dieser Systeme darstellt (H. Westlinning, Kautschuk, Gummi, Kunststoffe 23 (1970) 219; E. Morita, A.B. Sullivan, A.Y. Coran, Rubber Chem. Technol. 58 (1985) 284). Insbesondere Aminogruppen und polysulfidische Gruppen tragende Derivate sind interessante Alternativen zu den konventionellen Beschleunigern, da sie neben ihrem positiven Einfluß auf die Vulkanisation auch noch als Schwefelspender wirken (Ullmann's Encyclopedia of Industrial Chemistry, 4. Auflage, Bd. A23, S. 375).

Nachteil der bekannten Organosiliciumverbindungen ist, daß diese nicht gleichzeitig als gute Haftvermittler und gute Vulkanisationsbeschleuniger, Schwefelspender, Vernetzter oder Alterungsschutzmittel wirken.

Aufgabe der vorliegenden Erfindung ist es triazinfunktionelle Haftvermittler zur Verfügung zu stellen, die neben ihrer Funktion als Haftvermittler im Vulkanisat gleichzeitig noch als Vulkanisationsbeschleuniger, Schwefelspender, Vernetzer oder Alterungsschutzmittel wirken.

Gegenstand der Erfindung ist eine Organosiliciumverbindung der allgemeinen Formel I 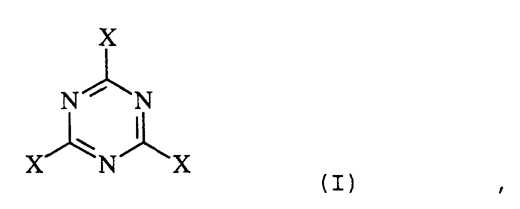 welche dadurch gekennzeichnet ist, dass die Substituenten X gleich oder verschieden sind und X eine der folgenden Gruppen A, B oder C ist:
A = Y-R₁-Sₙ- mit 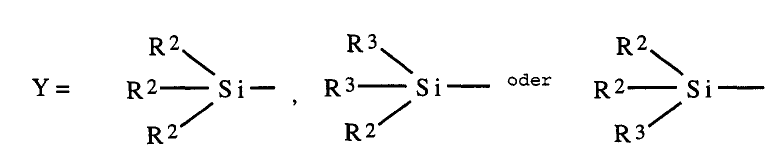 mit
   R² = Alkoxyrest mit 1 bis 4 C-Atomen,
   R³ = Alkylrest mit 1 bis 8 C-Atomen,
   R¹ = linear oder verzweigtem Alkyliden mit 1 bis 10 C-Atomen,
   n = 1 - 8 oder Gemische davon,
B = OR⁴, NR⁵R⁶, SR⁷, SCN oder -CO-R⁸ mit
   R⁴, R⁵, R⁶, R⁷ = H, verzweigter oder unverzweigter Alkylrest mit 1 - 10 C-Atomen oder substituierter oder unsubstituierter aromatischer Rest mit 6 - 30 C-Atomen, der gegebenenfalls durch N-, S- oder O-Atome unterbrochen ist,
   R⁸ = linear oder verzweigter Alkylrest mit 1-20 C-Atomen, bevorzugt Methyl oder langkettige, ungeradzahlige Alkylreste C₉ - C₁₇,
C = (Sₘ)/2 mit
   m = 1- 8 oder Gemische davon
   mit der Maßgabe, daß die Gruppe C zwei Triazin-Einheiten verbrückt,
und im Molekül mindestens eine Gruppe A vorhanden ist und die Kombination einer Gruppe A zusammen mit zwei Mercaptogruppen beziehungsweise einer Mercaptogruppe und einer Aminogruppe NR⁵R⁶ ausgeschlossen ist.

Entsprechend substituierte Triazinverbindungen können als Vernetzer zwischen Kautschukketten und Füllstoff wirken. Dabei kann mindestens ein Substituent mit dem Füllstoff und mindestens ein Substituent mit dem Polymer reagieren.

Entsprechende Substituenten können als Schwefelspender wirken.

Entsprechend substituierte Triazinverbindungen können als Vernetzer zwischen verschiedenen Kautschukketten wirken. Dabei können mindestens zwei Substituenten des Triazinmoleküls mit unterschiedlichen Kautschukketten reagieren und die Kautschukketten über die starre Triazineinheit verbinden.

Bei entsprechend substitutierten Triazinverbindungen kann ein Alterungsschutzmittel, das als Substituent an den Triazinring gebunden ist, in den Gummi eingebracht werden. Substanzen, die solche Wirkungen haben können, sind beispielsweise aromatische Amine und Phenole (Ullmann's Encyclopedia of Industrial Chemistry, 4. Auflage, Bd. 23, S. 383 ff).

Es ist bekannt, daß die drei Cl-Atome im Cyanurchlorid selektiv gegen Nucleophile ausgetauscht werden können (V.I. Mur, Russian Chem. Rev. 33 (1964) 92, Ullmann's Encyclopedia of Industrial Chem., 4. Auflage, Bd. A8, S. 195 f).

Ein weiterer Gegenstand ist ein Verfahren zur Herstellung von Organosiliciumverbindungen der Formel I, welches dadurch gekennzeichnet ist, dass man die Gruppe A erhält
- durch Umsetzung (II) eines chlorsubstituierten Triazingrundgerüsts mit Mercaptosilanen der entsprechenden Struktur in Gegenwart eines Säurefängers, beispielsweise tertiäre Amine, Alkalicarbonate oder durch Ausblasen des entstehenden HCl-Gases, wobei man abhängig von der Zahl der Chloratome am Triazingerüst und vom molaren Verhältnis Triazin : Mercaptosilan selektiv eine Mono-, Di- oder Trisubstitution erhält, oder 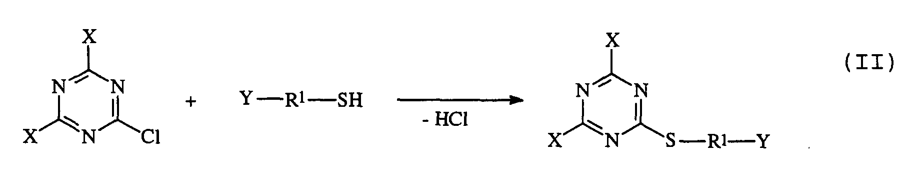
- durch Umsetzung (III) eines metallierten Mercaptotriazin mit einem der Gruppierung A entsprechendem Chloralkylsilan und für n > 1 unter Anwesenheit von elementarem Schwefel, 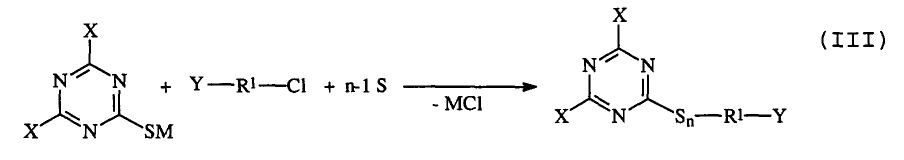 mit M = H, Metall, beispielsweise Na, K oder Li, die Gruppe B erhält
- durch Umsetzung (IV) eines chlorsubstituierten Triazingerüsts mit entsprechenden Alkoholen, Aminen, und Mercaptanen in Gegenwart eines Säurefängers, beispielsweise eines tertiären Amins (bei Reaktion mit einem Amin in Gegenwart eines Überschusses des gleichen Amins), Alkalicarbonaten oder durch Ausblasen des entsehenden HCl-Gases oder 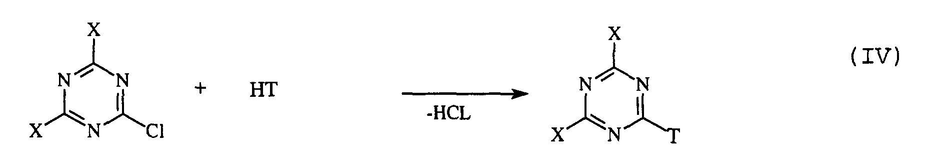 mit T = OR⁴, NR⁵R⁶ oder SR⁷,
- durch Umsetzung (V) eines chlorsubstituierten Triazingerüsts mit entsprechenden metallierten Alkoholen, Aminen, und Mercaptanen oder 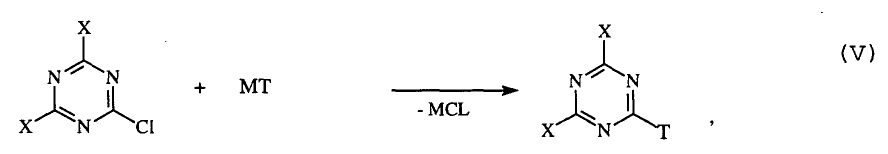
- durch Alkylierung (VI) entsprechender amino- und mercaptylsubstituierten Triazinen mit stark alkylierenden Substanzen (Z = I, Br, Cl, (SO₄)_{0,5}, 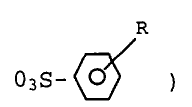 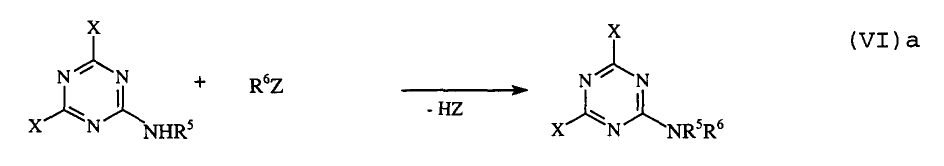 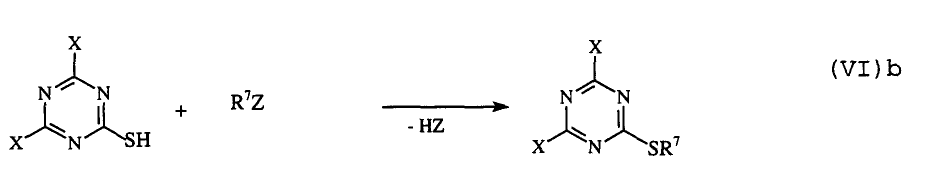 und die Gruppe C erhält
- durch Umsetzung eines chlorsubstituierten Triazins mit einem Natriumpolysulfid (VII) 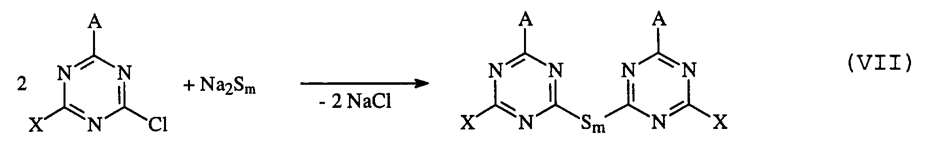 oder einem Gemisch von Natriumsulfid (VIIIa) beziehungsweise Natriumhydrogensulfid (VIIIb) und Schwefel 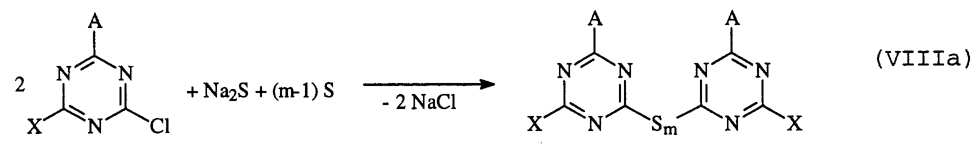 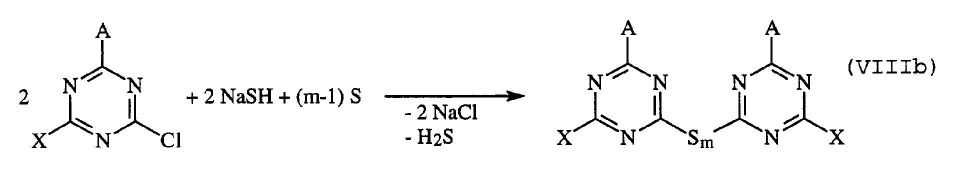
- durch Umsetzung (IX) eines Mercaptotriazins oder eines durch Metallierung aktivierten Mercaptotriazins (X) mit Schwefeldichloriden oder 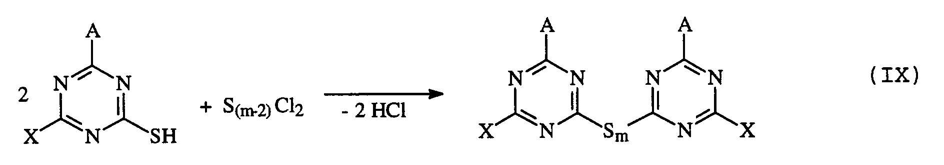 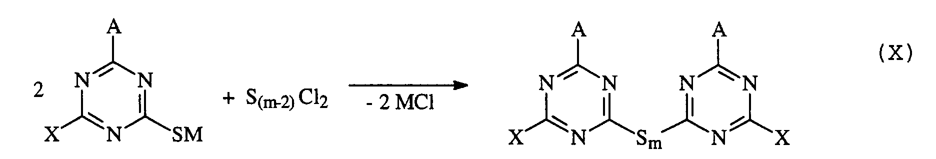
- durch Umsetzung (XI) eines Mercaptotriazins mit elementarem Schwefel bei erhöhter Temperatur oder 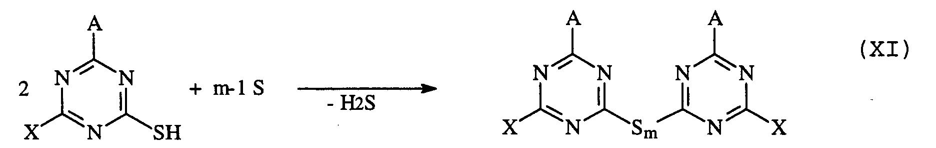
- durch Umsetzung (XII) eines durch Metallierung aktivierten Mercaptotriazins mit Schwefel und einem chlorsubstituiertem Triazinderivat

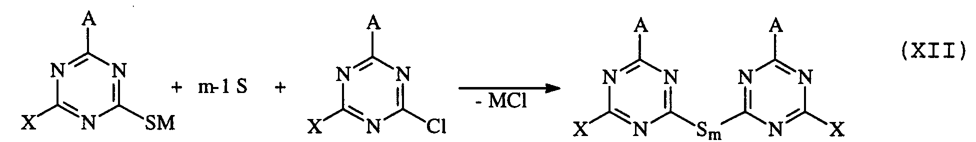

Die Reihenfolge der Umsetzung ist unerheblich. Bevorzugt kann die Gruppe C nach der Gruppe A eingeführt werden.

Ein weiterer Gegenstand der vorliegenden Erfindung sind Kautschukmischungen, welche dadurch gekennzeichnet sind, daß sie Kautschuk, Füllstoff, vorzugsweise gefällte Kieselsäure, mindestens eine Organosiliciumverbindung der Formel (I) und gegebenenfalls weitere Kautschukhilfsmittel enthalten.

Als Kautschuk kann Naturkautschuk und/oder Synthesekautschuke verwendet werden. Bevorzugte Synthesekautschuke sind beispielsweise bei W. Hofmann, Kautschuktechnologie, Genter Verlag, Stuttgart 1980, beschrieben. Die Kautschuke können sowohl alleine als auch in Kombination verwendet werden. Für die Herstellung von Kfz-Reifen können insbesondere anionisch polymerisierte L-SBR-Kautschuke mit einer Glastemperatur oberhalb von -50 °C sowie deren Mischungen mit hoch cis-haltigen Dienkautschuken eingesetzt werden.

Als Füllstoffe können eingesetzt werden:
- Ruße, die nach dem Flammruß-, Furnace- oder Gasruß-Verfahren hergestellt sind und BET-Oberflächen von 20 bis 200 m²/g besitzen,
- hochdisperse Kieselsäuren, hergestellt zum Beispiel durch Fällungen aus Silikatlösungen oder durch Flammenhydrolyse von Siliciumhalogeniden, mit spezifischen Oberflächen von 5 bis 1000 m²/g, vorzugsweise 20 bis 400 m²/g (BET-Oberfläche) und mit Primärteilchengrößen von 10 bis 400 nm, gegebenenfalls auch als Mischoxide mit anderen Metalloxiden, wie Al-, Mg-, Ca-, Ba-, Zn- und Titanoxiden,
- synthetische Silikate, wie Aluminiumsilikat, Erdalkalisilikate wie beispielsweise Magnesiumsilikat oder Calciumsilikat, mit BET-Oberflächen von 20 bis 400 m²/g und Primärteilchendurchmessern von 10 bis 400 nm,
- natürliche Silikate, wie Kaolin und andere natürlich vorkommende Kieselsäuren,
- Glasfasern und Glasfaserprodukte (Matten, Stränge) oder Mikroglaskugeln.

Die Kautschukmischungen können Synthesekautschuk und Kieselsäure als Füllstoff enthalten. Bevorzugt können hochdisperse Kieselsäuren, hergestellt durch Fällung aus Silikatlösungen, mit BET-Oberflächen von 20 bis 400 m²/g in Mengen von 10 bis 150 Gew.-Teilen, bezogen auf 100 Gew.- Teile Kautschuk, eingesetzt werden.

Die genannten Füllstoffe können alleine oder als Gemisch eingesetzt werden.

Die erfindungsgemäßen Organosiliciumverbindungen können sowohl in reiner Form als auch aufgezogen auf einen inerten organischen oder anorganischen Träger, verwendet werden. Bevorzugte Trägermaterialien können Kieselsäure, natürliche oder synthetische Silikate, Aluminiumoxid oder Ruße sein. Die erfindungsgemäßen Organosiliciumverbindungen können alleine oder in Kombination mit anderen Organosiliciumverbindungen, insbesondere monofunktionellen Alkylalkoxysilanen, verwendet werden.

Als Kautschukhilfsprodukte können Reaktionsbeschleuniger, Reaktionsverzögerer, Alterungsschutzmittel, Stabilisatoren, Verarbeitungshilfsmittel, Weichmacher, Wachse, Metalloxide sowie Aktivatoren, wie Triethanolamin, Polyethylenglykol, Hexantriol, die der Kautschukindustrie bekannt sind, verwendet werden.

Vorteil der erfindungsgemäßen Organosiliciumverbindungen ist, dass diese als Kopplungsreagenzien und bei geeigneten Substituenten am Triazinring auch als Beschleuniger oder Alterungs- beziehungsweise Ermüdungsschutzmittel wirken. Zudem sind die erfindungsgemäßen Organosiliciumverbindungen als Haftvermittler für Gummi-Metall- und Gummi-Faser-Verbindungen geeignet, insbesondere bei primären oder sekundären Aminosubstituenten am Triazinring.

### Beispiele:

### Beispiel 1: Herstellung von 1,3,5-Tris(triethoxysilylpropylmercaptyl)triazin aus Cyanurchlorid und 3-Mercaptopropyltriethoxysilan

36,6 g Cyanurchlorid werden in einem 1 1 Dreihalskolben mit Rückflußkühler, Innenthermometer und Tropftrichter bei 0°C mit 62,7 g Triethylamin versetzt. Zu diesem Gemisch werden 143,1 g Mercaptopropyltriethoxysilan unter Kühlung zugetropft. Sofort fällt ein weißer Niederschlag aus. Nach Beendigung der Zugabe wird weitere 2 h bei 20 - 25°C gerührt und anschließend 5 h unter Rückfluß erhitzt. Nach Abkühlen werden ausgefallenes Triethylammoniumchlorid durch Filtration abgetrennt, der Filterkuchen vier mal mit jeweils 75 ml Toluol gewaschen und die vereinigten Filtrate im Vakuum eingedampft. Nach erneuter Feststoffabtrennung erhält man 155,4 g eines gelben Öls. Mit ¹H-NMR wird 1,3,5-Tris(triethoxysilylpropylmercaptyl)triazin nachgewiesen.

### Beispiel 2: Herstellung von 1,3,5- Tris(triethoxysilylpropylmercaptyl)triazin aus dem Trinatriumsalz des 1,3,5-Trimercaptotriazins und 3-Chlorpropyltriethoxysilan

24,3 g des Trinatriumsalzes von 1,3,5-Trimercaptotriazin werden in 75 ml Ethanol suspendiert und mit 72,2 g Chlorpropyltriethoxysilan und 0,3 g Aliquat 336 versetzt. Das Gemisch wird in einem Autoklaven unter Rühren 5 h auf 140°C gehalten. Nach Abkühlen auf Raumtemperatur wird von gebildeten Natriumchlorid abfiltriert, der Niederschlag vier mal mit jeweils 20 ml Ethanol gewaschen und die vereinigten Filtrate im Vakuum eingedampft. Es wird erneut von wenig ausgefallenem Niederschlag abfiltriert. Man erhält 75,3 g eines gelben Öls. Mit ¹H-NMR wird 1,3,5-Tris(triethoxysilylpropylmercaptyl)triazin nachgewiesen.

### Beispiel 3: Herstellung von l-(Di(n-butyl)amino)-3-triethoxysilylpropylmercaptyl-5-(1-methoxypropyl)aminotriazin aus dem Natriumsalz des 1-(Di(n-butyl)amino)-3-mercaptyl-5-(1-methoxypropyl)aminotriazin und 3-Chlorpropyltriethoxysilan

17,47 g des Natriumsalzes des 1-(Di(n-butyl)amino)-3-mercaptyl-5-(1-methoxypropyl)aminotriazin wird in einem 250 ml-Dreihalskolben mit Rückflußkühler, Innenthermometer und Tropftrichter in 75 ml Ethanol gelöst. Zu diesem Gemisch läßt man bei Raumtemperatur 12,0 g 3-Chlorpropyltriethoxysilan zulaufen und erhitzt 3 h unter Rückfluß. Nach Abkühlen auf Raumtemperatur wird von gebildetem Natriumchlorid-Niederschlag abfiltriert und der Filterkuchen vier mal mit jeweils 20 ml Ethanol gewaschen. Die vereinigten Filtrate werden im Vakuum eingedampft. Man erhält 21,70 g eines gelben Öls. Mit ¹H-NMR wird 1-(Di(n-butyl)amino)-3-triethoxysilylpropylmercaptyl-5-(1-methoxypropyl)aminotriazin nachgewiesen.

### Beispiel 4: Herstellung von Bis(5,5'-(1-Dimethylamino-3-triethoxysilylpropylmercaptyltriazin)tetrasulfan aus 5-Chlor-1-Dimethylamino-3-triethoxysilylpropylmercaptyltriazin und Dinatriumtetrasulfid

Zu einer Lösung von 17,4 g Natriumtetrasulfid in 50 ml Wasser wird bei 95°C in Gegenwart eines Phasentransferkatalysators ein Gemisch aus 86,6 g 5-Chlor-1-Dimethylamino-3-triethoxysilylpropylmercaptyltriazin und 20 ml Toluol zugetropft. Die anfänglich rotorange wäßrige Phase entfärbt sich schnell. Nach 60 min Reaktionszeit wird die organische Phase abgetrennt und im Vakuum eingedampft. Man erhält 89 g Bis(5,5'-(1-Dimethylamino-3-triethoxysilylpropylmercaptyltriazin)tetrasulfan, dessen Identität mittels ¹H-NMR-Spektroskopie bestätigt wird.

### Beispiel 5:

### Kautschukmischungen

Die für die Herstellung der Kautschukmischungen verwendete Rezeptur ist in Tabelle 1 angegeben. Dabei bedeutet die Einheit phr Gewichtsanteile bezogen auf 100 Teile des eingesetzten Rohkautschuks.

**Tabelle 1**

| Substanz | Vergleichsbeispiel Menge [phr] | Beispiel B1 Menge [phr] | Beispiel B2 Menge [phr] |
|---|---|---|---|
| 1. Stufe | | | |
| Buna VSL 5025-1 | 96,0 | 96,0 | 96,0 |
| Buna CB 24 | 30,0 | 30,0 | 30,0 |
| Ultrasil 7000 GR | 80,0 | 80,0 | 80,0 |
| ZnO | 3,0 | 3,0 | 3,0 |
| Stearinsäure | 2,0 | 2,0 | 2,0 |
| Naftolen ZD | 10,0 | 10,0 | 10,0 |
| Vulkanox 4020 | 1,5 | 1,5 | 1,5 |
| Protector G35P | 1,0 | 1,0 | 1,0 |
| Bis(triethoxysilyl-propyl) disulfan | 5,8 | - | - |
| Silan der Formel (XIII) | - | 5,0 | 6,1 |
| | | | |

| 2. Stufe | | | |
|---|---|---|---|
| Batch Stufe 1 | | | |
| 3. Stufe | | | |
| Batch Stufe 2 | | | |
| Vulkacit D | 1,5 | 1,5 | 1,5 |
| Vulkacit CZ | 1,5 | 1,5 | 1,5 |
| Schwefel | 2,1 | 2,1 | 2,1 |

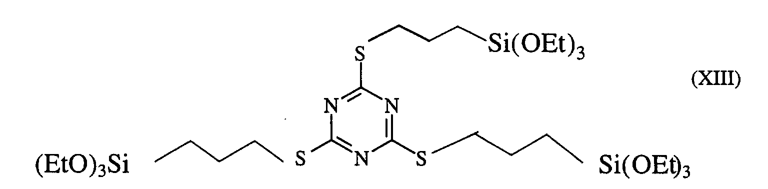

Bei dem Polymer VSL 5025-1 handelt es sich um ein in Lösung polymerisiertes SBR-Copolymer der Bayer AG mit einem Styrolgehalt von 25 Gew.-% und einem 1,2-Butadiengehalt von 50 %. Das Copolymer enthält zudem 37,5 phr Öl.

Bei dem Polymer Buna CB 24 handelt es sich um ein cis 1,4 Polybutadien (Neodymtyp) der Bayer AG mit cis 1,4-Gehalt von 97 %, einem trans 1,4-Gehalt von 2 %, einem 1,2-Gehalt von 1 %.

Die Kieselsäure Ultrasil 7000 GR der Degussa-Hüls AG besitzt eine BET-Oberfläche von 175 m²/g. Das Bis(triethoxysilylpropyl)disulfan hat einen Disulfangehalt von 85 %.

Als aromatisches Öl wird Naftolen ZD der Firma Chemetall verwendet; bei Vulkanox 4020 handelt es sich um PPD der Bayer AG und Protektor G35P ist ein Ozonschutzwachs der HB-Fuller GmbH. Vulkacit D (DPG) und Vulkacit CZ (CBS) sind Handelsprodukte der Bayer AG.

Die Kautschukmischungen werden dreistufig in einem Innenmischer entsprechend der folgenden tabellarischen Aufstellung (Tabelle 2) hergestellt:

**Tabelle 2:**

| **Stufe 1** | | |
|---|---|---|
| **Einstellungen** | | |
| Mischaggregat | | Werner & Pfleiderer E-Typ |
| Friktion | | 1:1 |
| Drehzahl | | 70 min⁻¹ |
| Stempeldruck | | 5,5 bar |
| Leervolumen | | 1,6 L |
| Füllgrad | | 0,55 |
| Durchflußtemp. | | 80 °C |

| **Mischvorgang** | | |
|---|---|---|
| 0 bis | 1 min | Buna VSL 5025-1 + Buna CB 24 |
| 1 bis | 3 min | ½ Ultrasil 7000 GR, ZnO, Stearinsäure, |
| | | Naftolen ZD, Silan |
| 3 bis | 4 min | ½ Ultrasil 7000 GR, Vulkanox 4020, |
| | | Protector G35P |
| | 4 min | Säubern |
| 4 bis | 5 min | Mischen |
| | 5 min | Säubern |
| 5 bis | 6 min | Mischen und ausfahren |
| | | |
| Batch-Temp. | | 140-150°C |
| Lagerung | | 24 h bei Raumtemperatur |

| **Stufe 2** | | |
|---|---|---|
| **Einstellungen** | | |
| Mischaggregat | | Wie in Stufe 1 bis auf: |
| Drehzahl | | 80 min⁻¹ |
| Füllgrad | | 0,53 |
| Durchflußtemp. | | 80 °C |

| **Mischvorgang** | | |
|---|---|---|
| 0 bis | 2 min | Batch Stufe 1 aufbrechen |
| 2 bis | 5 min | Batchtemperatur 150°C durch Drehzahlvariation halten |
| | 5 min | Ausfahren |
| | | |
| Batch-Temp. | | 150-155°C |
| Lagerung | | 4 h bei Raumtemperatur |

| **Stufe 3** | | |
|---|---|---|
| **Einstellungen** | | |
| Mischaggregat | | Wie in Stufe 1 bis auf: |
| Drehzahl | | 40 min⁻¹ |
| Füllgrad | | 0,51 |
| Durchflußtemp. | | 50 °C |

| **Mischvorgang** | | |
|---|---|---|
| 0 bis 2 min | | Batch Stufe 2 + Vulkacit CZ + Vulkacit D + Schwefel |
| 2 min | | Ausfahren und auf Labormischwalzwerk Fell bilden |
| | | Durchflußtemperatur 50°C) |
| | | |
| | | Homogenisieren: |
| | | 3* links, 3* rechts einschneiden und umklappen sowie |
| | | 8* bei engem Walzenspalt (1 mm) und |
| | | 3* bei weitem Walzenspalt (3,5 mm) stürzen und |
| | | Anschließend ein Fell ausziehen |
| | | |
| Batch-Temp. | | 85-95°C |

Das allgemeine Verfahren zur Herstellung von Kautschukmischungen und deren Vulkanisate ist in "Rubber Technology Handbook", W. Hofmann, Hanser Verlag 1994 beschrieben.

Die Vulkanisationszeit für die Prüfkörper beträgt 30 Minuten bei 165°C.

Die gummitechnische Prüfung erfolgt gemäß den in Tabelle 3 angegebenen Prüfmethoden.

**Tabelle 3**

| Physikalische Testung | Norm/ Bedingungen |
|---|---|
| ML 1+4, 100°C | DIN 53523/3, ISO 667 |
| Vulkameterprüfung, 165°C | DIN 53529/3, ISO 6502 |
| Zugversuch am Ring, 23°C | DIN 53504, ISO 37 |
| Zugfestigkeit | |
| Spannungswerte | |
| Bruchdehnung | |
| Shore-A-Härte, 23°C | DIN 53 505 |
| Viskoelastische Eigenschaften, | DIN 53 513, ISO 2856 |
| 0 und 60°C, 16 Hz, 50 N Vorkraft | |
| und 25 N Amplitudenkraft | |
| Komplexer Modul E*, | |
| Verlustfaktor tan δ | |
| Ball-Rebound | |
| DIN-Abrieb, 10 N Kraft | DIN 53 516 |
| Dispersion | DIN/ISO 11 345 |
| Mooney-Scorch, 130 °C t5, t 35 | DIN 53523, ISO 667 |

Tabelle 4 zeigt die gummitechnischen Daten.

**Tabelle 4:**

| **Mischung** | | **-1-** Vergleichsbeispiel | **-2-** Beispiel B1 | Beispiel B2 |
|---|---|---|---|---|
| **Rohmischungsergebnisse:** | | | | |
| ML 1+4 (3.Mischstufe) | [ME] | 61 | 64 | 64 |
| Mooney-Scorch (135 °C) t5 | [min] | >60 | >60 | >60 |
| t10% bei 165°C | [min] | 2,7 | 1,1 | 1,4 |
| t90% bei 165°C | [min] | 25,2 | 28,7 | 21,5 |
| Dmax-Dmin bei 165°C | [dNm] | 17,3 | 20,5 | 18,5 |

| **Vulkanisatergebnisse:** | | | | |
|---|---|---|---|---|
| Shore-A-Härte | [SH] | 66 | 67 | 67 |
| Zugfestigkeit | [MPa] | 13,1 | 13,7 | 14,4 |
| Spannungswert 100 % | [MPa] | 1,9 | 1,8 | 1,8 |
| Spannungswert 300 % | [MPa] | 8,9 | 7,8 | 8,2 |
| Spannungswert 300 %/100 % | [-] | 4,6 | 4,3 | 4,5 |
| Bruchdehnung | [%] | 380 | 440 | 440 |
| Bruchenergie | [J] | 66,4 | 81,2 | 87,5 |
| Ball-Rebound 0°C | [%] | 10,5 | 11,0 | 10,6 |
| Ball-Rebound 60°C | [%] | 59,4 | 57,1 | 56,6 |
| **DIN-Abrieb** | [mm³] | 87,7 | 101,7 | 100,4 |
| Dyn. Dehnmodul E'(0°C) | [MPa] | 20,4 | 21,1 | 24,9 |
| Dyn. Dehnmodul E'(60°C) | [MPa] | 7,7 | 7,6 | 8,1 |
| Dyn. Dehnmodul E"(0°C) | [MPa] | 10,6 | 11,1 | 13,2 |
| Dyn. Dehnmodul E"(60°C) | [MPa] | 1,1 | 1,1 | 1,2 |
| Verlustfaktor tan δ(0°C) | [-] | 0,518 | 0,529 | 0,531 |
| Verlustfaktor tan δ(60°C) | [-] | 0,137 | 0,151 | 0,152 |
| Dispersion | [-] | 8 | 7 | 7 |

Die Beispiele zeigen, dass eine Füllstoff-Kautschukanbindung erfolgt ist.

Bei equimolarer Dosierung (6,1 phr) belegen die statischen als auch dynamischen Daten eine Polymeranbindung.

### Beispiel 6: Herstellung von 1,3-Diethoxy-5-triethoxysilylpropylmercaptyltriazin aus 1,3-Diethoxy-5-mercaptotriazin

Zu einer Lösung bestehend aus 9,1 g (0,4 mol) Natrium in 300 ml Ethanol werden bei 50°C 80,0 g (0,4 mol) 1,3-Diethoxy-5-mercaptotriazin in 200 ml Ethanol gegeben. Nach 20 Minuten bei 50°C werden 95,7 g (0,4 mol) Chlorpropyltriethoxysilan zugetropft. Anschließend wird 6 h lang bei 78°C gerührt. Nach Abkühlen auf Raumtemperatur wird der ausgefallende Niederschlag abfiltriert und das Lösungsmittel (Ethanol) am Rotationsverdampfer entfernt. Man erhält 121,7 g 1,3-Diethoxy-5-triethoxysilylpropylmercaptyltriazin, dessen Identität mittels ¹H-NMR-Spektroskopie bestätigt wird.

### Beispiel 7:

### Kautschukmischungen

Die für die Herstellung der Kautschukmischungen verwendete Rezeptur ist in Tabelle 5 angegeben. Das Silan des Beispiels B3 wird equimolar und das Silan des Beispiels B4 wird gewichtsgleich, bezogen auf das Silan der Referenzmischung, eingesetzt.

**Tabelle 5**

| Substanz | Vergleichsbeispiel Menge [phr] | Beispiel B3 Menge [phr] | Beispiel B4 Menge [phr] |
|---|---|---|---|
| 1. Stufe | | | |
| Buna VSL 5025-1 | 96,0 | 96,0 | 96,0 |
| Buna CB 24 | 30,0 | 30,0 | 30,0 |
| Ultrasil 7000 GR | 80,0 | 80,0 | 80,0 |
| ZnO | 3,0 | 3,0 | 3,0 |
| Stearinsäure | 2,0 | 2,0 | 2,0 |
| Naftolen ZD | 10,0 | 10,0 | 10,0 |
| Vulkanox 4020 | 1,5 | 1,5 | 1,5 |
| Protector G35P | 1,0 | 1,0 | 1,0 |
| Bis(triethoxysilyl- | 6,4 | - | - |
| propyl)tetrasulfan(Si69) | | | |
| Silan gemäß Beispiel 6 | - | 9,74 | 6,4 |
| | | | |

| 2. Stufe | | | |
|---|---|---|---|
| Batch Stufe 1 | | | |
| 3. Stufe | | | |
| Batch Stufe 2 | | | |
| Vulkacit D | 1,5 | 1,5 | 1,5 |
| Vulkacit CZ | 1,5 | 1,5 | 1,5 |
| Schwefel | 1,5 | 2,2 | 2,2 |

Die Kautschukmischungen werden dreistufig, wie in Beispiel 5, Tabelle 2, hergestellt.

Die Vulkanisationszeit für die Prüfkörper beträgt 30 Minuten für das Vergleichsbeispiel und Beispiel B3 und für Beispiel B4 45 Minuten bei 165°C.

Die gummitechnische Prüfung erfolgt gemäß dem Beispiel 5, Tabelle 3 angegebenen Prüfmethoden.

Tabelle 6 zeigt die gummitechnischen Daten.

**Tabelle 6:**

| **Mischung** | | Vergleichs -beispiel -Si 69 Ref- | Beispiel B3 -1- equimolar | Beispiel B4 -2- gew.gleich |
|---|---|---|---|---|
| **Rohmischungsergebnisse:** | | | | |
| ML(1+4) bei 100°C, | [ME] | 64 | 52 | 58 |
| 3. Stufe | | | | |
| Scorch-Zeit, t5 (135 °C) | [min] | 38,9 | 57,5 | 56,5 |
| Scorch-Zeit, t35 (135 °C) | [min] | 54,3 | >60 | >60 |
| Dmax-Dmin bei 165°C | [dNm] | 16,9 | 16,4 | 20,7 |
| t10% bei 165°C | [min] | 1,8 | 4,9 | 3,6 |
| t90% bei 165°C | [min] | 19,5 | 28,2 | 50,0 |

| **Vulkanisatergebnisse:** | | | | |
|---|---|---|---|---|
| Zugfestigkeit | [MPa] | 11,0 | 10,6 | 12,3 |
| Spannungswert 100 % | [MPa] | 1,7 | 1,4 | 1,6 |
| Spannungswert 300 % | [MPa] | 9,1 | 6,1 | 6,9 |
| Spannungswert 300 %/100 % | [-] | 5,2 | 4,3 | 4,3 |
| Bruchdehnung | [%] | 340 | 440 | 450 |
| Bruchenergie | [J] | 46,7 | 63,8 | 75,8 |
| Shore-A-Härte | [SH] | 64 | 63 | 66 |
| Ball-Rebound,23°C | [%] | 32,4 | 22,2 | 25,0 |
| Speichermodul E',0°C | [MPa] | 16,6 | 27,5 | 23,8 |
| Speichermodul E',60°C | [MPa] | 7,1 | 6,6 | 7,4 |
| Verlustmodul E", 0°C | [MPa] | 8,4 | 16,4 | 13,4 |
| Verlustmodul E", 60°C | [MPa] | 1,0 | 1,2 | 1,3 |
| Verlustfaktor tan δ(0°C) | [-] | 0,508 | 0,596 | 0,563 |
| Verlustfaktor tan δ(60°C) | [-] | 0,135 | 0,182 | 0,172 |
| Dispersion | [-] | 9 | 9 | 9 |

Die Beispiele B3 und B4 (Silan gemäß Beispiel 6)zeigen eine Kautschuk-Füllstoff-Kopplungswirkung, längere Scorchzeit und niedrigere Viskosität gegenüber dem Vergleichsbeispiel.

### Beispiel 8: Herstellung von Bis-[2-diethylamino-4-(3-triethoxysilylpropyl)mercapto-s-triazin-6-yl]-polysulfid (XIV)

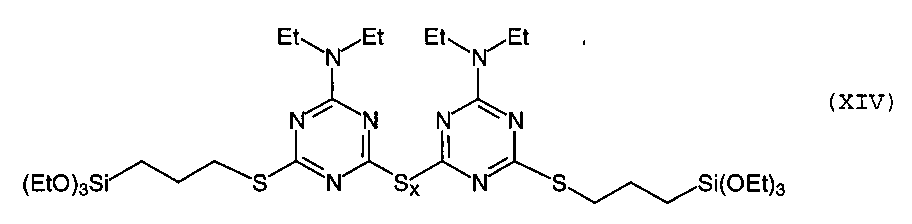

Zu einer Lösung aus 108,4 g 2-Diethylamino-4,6-dichlor-s-triazin und 58,0 g Triethylamin in 500 ml Toluol werden bei 10 °C 116,8 g Mercaptopropyltriethoxysilan getropft. Anschließend wird 1 h lang bei Raumtemperatur gerührt. Der ausgefallene Niederschlag wird abfiltriert und das Lösungsmittel (Toluol) am Rotationsverdampfer entfernt. Man erhält 213,6 g flüssiges Produkt, das zu einer Suspension aus 45,7 g Natriumpolysulfid (Na₂S_{3,8}) in 500 ml Ethanol bei Raumtemperatur getropft wird und anschließend 2 h lang bei 80 °C unter Rückfluß gerührt wird. Nach Abkühlen auf Raumtemperatur wird vom Niederschlag abfiltriert und das Filtrat am Rotationsverdampfer vom Ethanol befreit. Man erhält 185,8 g eines wachsartigen Feststoffs, dessen Identität mittels ¹H- und ¹³C-NMR-Spektroskopie bestätigt wird. Die mittlere Schwefelkettenlänge x beträgt 3,8.

### Beispiel 9

### Kautschukmischungen

Die für die Herstellung der Kautschukmischungen verwendete Rezeptur ist in Tabelle 7 angegeben.

**Tabelle 7**

| **Substanz** | **Vergleichs- beispiel I** **Menge [phr]** | **Beispiel B5** **Menge [phr]** |
|---|---|---|
| **1. Stufe** | | |
| Buna VSL 5025-1 | 96,0 | 96,0 |
| Buna CB 24 | 30,0 | 30,0 |
| Ultrasil 7000 GR | 80,0 | 80,0 |
| ZnO | 3,0 | 3,0 |
| Stearinsäure | 2,0 | 2,0 |
| Naftolen ZD | 10,0 | 10,0 |
| Vulkanox 4020 | 1,5 | 1,5 |
| Protector G35P | 1,0 | 1,0 |
| Bis(triethoxysilyl- | 6,4 | - |
| propyl)tetrasulfan(Si69) | | |
| Silan gemäß Beispiel 8 | - | 10,8 |
| | | |

| 2. Stufe | | |
|---|---|---|
| Batch Stufe 1 | | |
| 3. Stufe | | |
| Batch Stufe 2 | | |
| Vulkacit D | 1,5 | 1,5 |
| Vulkacit CZ | 1,5 | 1,5 |
| Schwefel | 1,5 | 1,5 |

Das Silan gemäß des Beispiels 8 wird in Beispiel B5 equimolar, bezogen auf die Triethoxysilylfunktion, zu dem Vergleichsbeispiel I mit Si 69 dosiert, was einer Menge von 10,8 phr entspricht.

In allen Mischungen wird 1,5 phr Schwefel zugegeben und gemäß der Mischvorschrift aus Beispiel 5, Tabelle 2 gearbeitet.

Die Vulkanisationszeit für die Prüfkörper beträgt 20 Minuten bei 165°C.

Die gummitechnische Prüfung erfolgt gemäß dem in Beispiel 5, Tabelle 3 angegebenen Prüfmethoden.

Tabelle 8 zeigt die gummitechnischen Daten.

**Tabelle 8:**

| **Mischung** | | **Vergleichs- beispiel I -Si 69 Ref-** | **Beispiel B 5 equimolar** |
|---|---|---|---|
| **Rohmischungsergebnisse** | | | |
| ML(1+4) bei 100°C, | | 67 | 66 |
| 3. Stufe | [ME] | | |
| Dmax-Dmin bei 165°C | [dNm] | 16,7 | 21,1 |
| t 10 % | [min] | 1,6 | 1,0 |
| t 90 % | [min] | 7,4 | 13,3 |

| **Vulkanisatergebnisse** | | | |
|---|---|---|---|
| Zugfestigkeit | [MPa] | 11,4 | 10,5 |
| Spannungswert 100 % | [MPa] | 1,7 | 3,5 |
| Spannungswert 300 % | [MPa] | 8,8 | - |
| Spannungswert 300 %/100 % | [-] | 5,2 | - |
| Bruchdehnung | [%] | 350 | 210 |
| Bruchenergie | [J] | 50 | 30 |
| Shore-A-Härte | [SH] | 62 | 73 |
| DIN-Abrieb | [mm³] | 79 | 66 |
| Ball-Rebound, 23°C | [%] | 33,6 | 33,3 |
| Komplexer Modul E*, 0°C | [MPa] | 17,2 | 27,7 |
| Komplexer Modul E*, 60°C | [MPa] | 7,5 | - |
| Verlustfaktor tan δ 0°C | [-] | 0,484 | 0,440 |
| Verlustfaktor tan δ 60°C | [-] | 0,121 | - |
| Dispersion Phillips | [-] | 7 | 7 |

Wie man anhand der Daten in Tabelle 8 erkennt, führt die equimolare Dosierung des erfindungsgemäßen Silans (Beispiel B5) zu einer hohen Vernetzungsdichte, die sich in einem sehr hohen Dₘₐₓ-Dₘᵢₙ-Wert, einer hohen Härte, hohen Moduli und kurzen Bruchdehnungen bemerkbar macht. Diese gesteigerte Vernetzungsdichte ist neben der hohen Kopplungsausbeute auch auf die Schwefelspenderfunktion der Polysulfanfunktion zurückzuführen.

### Beispiel 10:

### Kautschukmischungen

Die für die Herstellung der Kautschukmischungen verwendete Rezeptur ist in Tabelle 9 angegebenen.

**Tabelle 9**

| Substanz | Vergleichsbeispiel II Menge [phr] | Beispiel B6 Menge [phr] |
|---|---|---|
| 1. Stufe | | |
| Buna VSL 5025-1 | 96,0 | 96,0 |
| Buna CB 24 | 30,0 | 30,0 |
| Ultrasil 7000 GR | 80,0 | 80,0 |
| ZnO | 3,0 | 3,0 |
| Stearinsäure | 2,0 | 2,0 |
| Naftolen ZD | 10,0 | 10,0 |
| Vulkanox 4020 | 1,5 | 1,5 |
| Protector G35P | 1,0 | 1,0 |
| Bis(triethoxysilyl- | 6,4 | - |
| propyl)tetrasulfan(Si69) | | |
| Silan gemäß Beispiel 8 | - | 6,4 |
| Octyltriethoxysilan (Si 208) | | 2,0 |

| 2. Stufe | | |
|---|---|---|
| Batch Stufe 1 | | |
| 3. Stufe | | |
| Batch Stufe 2 | | |
| | | |
| Vulkacit D | 1,5 | 1,5 |
| Vulkacit CZ | 1,5 | 1,5 |
| Schwefel | 1,5 | 1,5 |

In Beispiel B6 wird das Silan aus Beispiel 8 gewichtsgleich (6,4 phr) zu dem Vergleichsbeispiel II dosiert. Um die geringere Hydrophobierung dieses Silans bei der gewichtsgleichen Dosierung, entsprechend einer geringeren Stoffmenge, zu kompensieren, wird zusätzlich 2 phr des monofunktionellen Alkylsilans Octyltriethoxysilan (Si 208) zugegeben. Eine Reaktion von Si 208 mit dem Kautschuk ist ausgeschlossen.

In allen Mischungen wird 1,5 phr Schwefel zugegeben und gemäß der Mischvorschrift aus Beispiel 5, Tabelle 2 gearbeitet.

Die Vulkanisationszeit für die Prüfkörper beträgt 20 Minuten bei 165°C.

Die gummitechnische Prüfung erfolgt gemäß dem in Beispiel 5, Tabelle 3 angegebenen Prüfmethoden.

Tabelle 10 zeigt die gummitechnischen Daten.

**Tabelle 10:**

| **Mischung** | | Vergleichsbeispiel II -Si 69 Ref- | Beispiel B 6 Gewichtsgleich |
|---|---|---|---|
| **Rohmischungsergebnisse** | | | |
| ML(1+4) bei 100°C, | | 65 | 60 |
| 3. Stufe | [ME] | | |
| Dmax-Dmin bei 165°C | [dNm] | 16,0 | 15,6 |
| t 10 % | [min] | 1,8 | 2,5 |
| t 90 % | [min] | 17,7 | 21,9 |

| **Vulkanisatergebnisse** | | | |
|---|---|---|---|
| Zugfestigkeit | [MPa] | 11,6 | 11,2 |
| Spannungswert 100 % | [MPa] | 1,8 | 1,8 |
| Spannungswert 300 % | [MPa] | 9,1 | 9,8 |
| Spannungswert 300 %/100 % | [-] | 5,1 | 5,4 |
| Bruchdehnung | [%] | 350 | 330 |
| Bruchenergie | [J] | 53 | 46 |
| Shore-A-Härte | [SH] | 63 | 62 |
| DIN-Abrieb | [mm³] | 81 | 84 |
| Ball-Rebound, 23°C | [%] | 33,9 | 31,8 |
| Komplexer Modul E*, 0°C | [MPa] | 12,5 | 13,0 |
| Komplexer Modul E*, 60°C | [MPa] | 6,6 | 6,6 |
| Verlustfaktor tan δ 0°C | [-] | 0,459 | 0,472 |
| Verlustfaktor tan δ 60°C | [-] | 0,129 | 0,113 |
| Dispersion Phillips | [-] | 8 | 7 |

In Beispiel B6 wird gewichtsgleich dosiert und die zu erwartende höhere Viskosität bei dem geringeren Silaneinsatz durch eine Zudosierung des Hydrophobierungshilfsmittels Octyltriethoxysilan Si 208 kompensiert. Man erkennt für Beispiel B6 eine vorteilhaft niedrige Viskosität, hohe Moduli, ein hohes Verstärkungsverhältnis 300 %/100 %, sowie ein vorteilhaft niedriger tan δ (60°C)-Wert, der auf eine sehr gute Füllstoff-Polymeranbindung schließen lässt.

## Patentansprüche

1. Organosiliciumverbindung der allgemeinen Formel I, 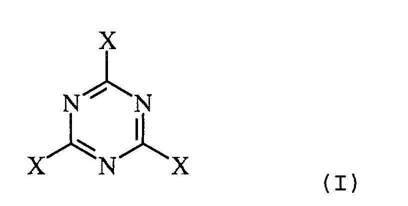 **dadurch gekennzeichnet, dass** die Substituenten X gleich oder verschieden sind und X eine der folgenden Gruppen A, B oder C ist:
A = Y-R₁-Sₙ- mit 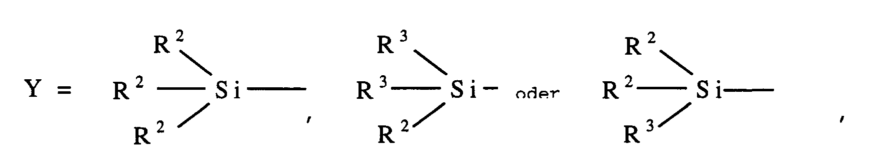 mit
R² = Alkoxyrest mit 1 bis 4 C-Atomen,
R³ = Alkylrest mit 1 bis 8 C-Atomen,
R¹ = linear oder verzweigtem Alkyliden mit 1 bis 10 C-Atomen.
n = 1 - 8 oder Gemische davon,
B = OR⁴, NR⁵R⁶, SR⁷, SCN oder -CO-R⁸ mit
R⁴, R⁵, R⁶, R⁷ = H, verzweigter oder unverzweigter Alkylrest mit 1 - 10 C-Atomen oder substituierter oder unsubstituierter aromatischer Rest mit 6 - 30 C-Atomen, der gegebenenfalls durch N-, S- oder O-Atome unterbrochen ist,
R⁸= linear oder verzweigter Alkylrest mit 1 - 20 C-Atomen,
C = (Sₘ)/2 mit
m = 1- 8 oder Gemische davon
mit der Maßgabe, daß die Gruppe C zwei Triazin-Einheiten verbrückt,
und im Molekül mindestens eine Gruppe A vorhanden ist und die Kombination einer Gruppe A zusammen mit zwei Mercaptogruppen beziehungsweise einer Mercaptogruppe und einer Aminogruppe NR⁵R⁶ ausgeschlossen ist.

2. Verfahren zur Herstellung von Organosiliciumverbindungen nach Anspruch 1, **dadurch gekennzeichnet, dass** man die Gruppe A erhält
• durch Umsetzung (II) eines chlorsubstituierten Triazingrundgerüsts mit Mercaptosilanen der entsprechenden Struktur in Gegenwart eines Säurefängers, beispielsweise tertiäre Amine, Alkalicarbonate oder durch Ausblasen des entstehenden HCl-Gases, wobei man abhängig von der Zahl der Chloratome am Triazingerüst und vom molaren Verhältnis Triazin : Mercaptosilan selektiv eine Mono-, Di- oder Trisubstitution erhält, oder 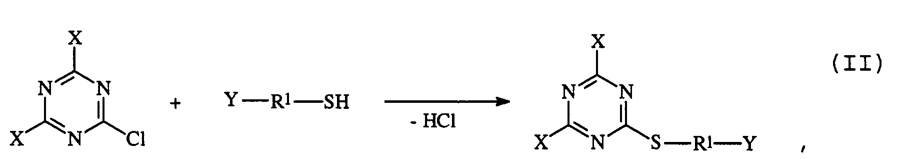
• durch Umsetzung (III) eines metallierten Mercaptotriazin mit einem der Gruppierung A entsprechendem Chloralkylsilan und für n > 1 unter Anwesenheit von elementarem Schwefel, 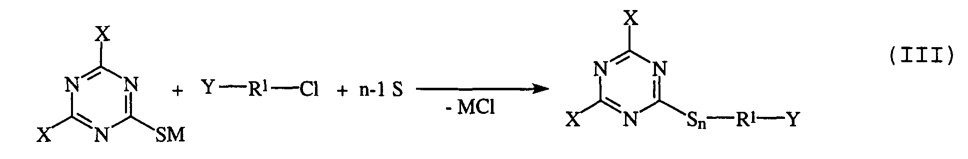 mit M = H, Metall,
die Gruppe B erhält
• durch Umsetzung (IV) eines chlorsubstituierten Triazingerüsts mit entsprechenden Alkoholen, Aminen, und Mercaptanen in Gegenwart eines Säurefängers, beispielsweise eines tertiären Amins (bei Reaktion mit einem Amin in Gegenwart eines Überschusses des gleichen Amins), Alkalicarbonaten oder durch Ausblasen des entsehenden HCl-Gases oder 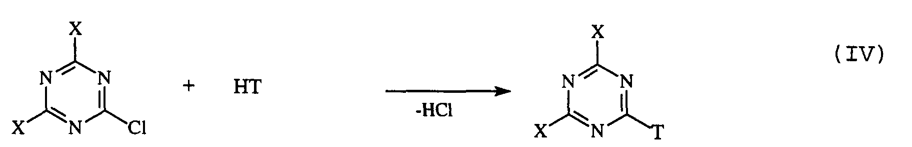 mit T = OR⁴, NR⁵R⁶ oder SR⁷,
• durch Umsetzung (V) eines chlorsubstituierten Triazingerüsts mit entsprechenden metallierten Alkoholen, Aminen, und Mercaptanen oder 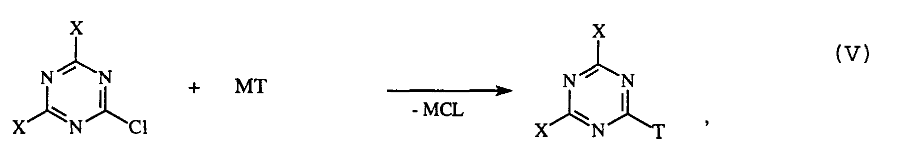
• durch Alkylierung (VI) entsprechender amino- und mercaptylsubstituierten Triazinen mit stark alkylierenden Substanzen 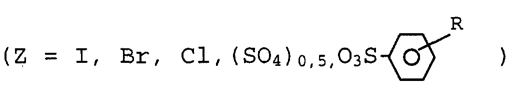 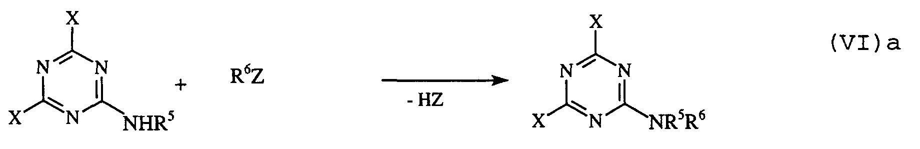 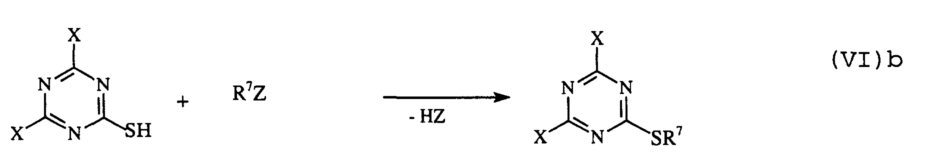 und die Gruppe C erhält
• durch Umsetzung (VII) eines chlorsubstituierten Triazins mit einem Natriumpolysulfid 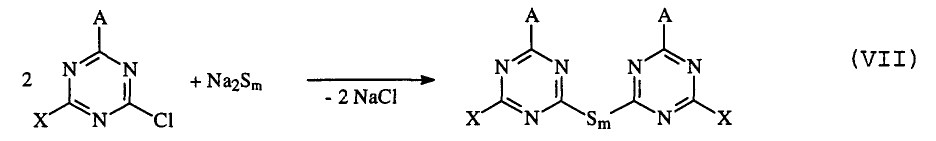 oder einem Gemisch von Natriumsulfid (VIIIa)
beziehungsweise Natriumhydrogensulfid (VIIIb) und Schwefel 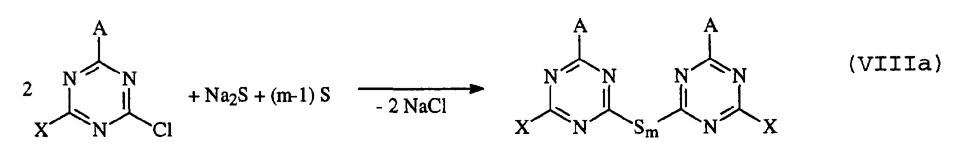 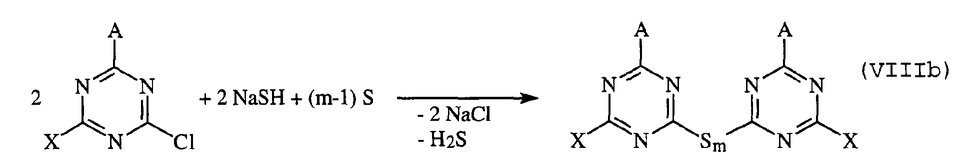
• durch Umsetzung (IX) eines Mercaptotriazins oder eines durch Metallierung aktivierten Mercaptotriazins (X) mit Schwefeldichloriden oder 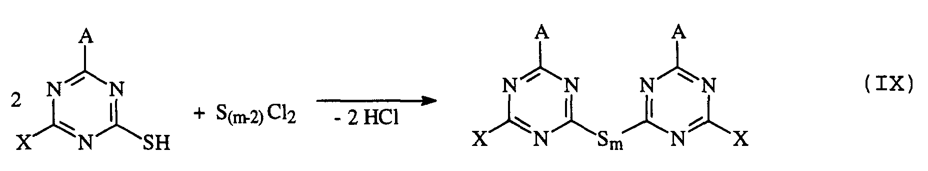 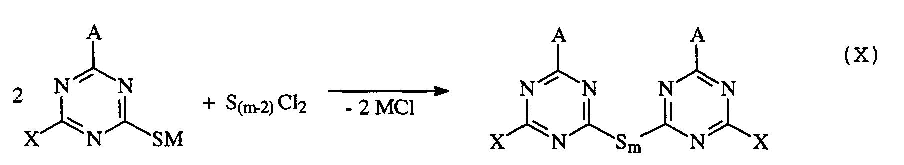
• durch Umsetzung (XI) eines Mercaptotriazins mit elementarem Schwefel bei erhöhter Temperatur oder 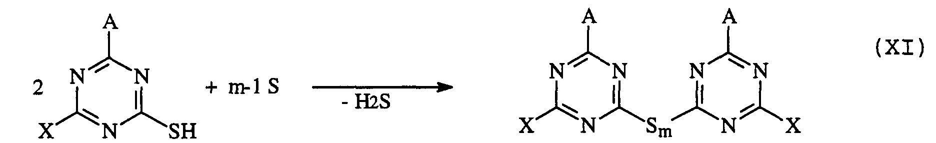
• durch Umsetzung (XII) eines durch Metallierung aktivierten Mercaptotriazins mit Schwefel und einem chlorsubstituiertem Triazinderivat

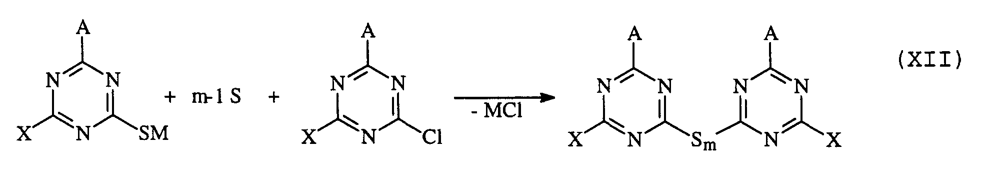

3. Verwendung der Organosiliciumverbindung nach Anspruch 1 in Kautschukmischungen.

4. Kautschukmischungen, **dadurch gekennzeichnet, daß** sie Kautschuk, Füllstoff, mindestens eine Organosiliciumverbindung der Formel (I) und gegebenenfalls weitere Kautschukhilfsmittel enthalten.
